# EUROPEAN PATENT APPLICATION

(11) **EP 1 813 283 A1**
(43) Date of publication of application: **01.08.2007**
(21) Application number: 05803349.9
(22) Date of filing: 02.11.2005
(51) Int. Cl.: A61K 45/00, A61K 31/485, A61K 31/495, A61K 38/00, A61P 25/04

(54) **REMEDY FOR NEUROPATHIC PAIN**

(30) Priority: 05.11.2004 JP 2004322072
(71) Applicant: Japan Science and Technology Agency, Kawaguchi-shi, Saitama 332-0012 (JP); National Universiy Corporation Tokyo Medical and Dental University, Tokyo 113-8510 (JP)
(72) Inventor: TANABE, Tsutomu, 1510053 (JP)
(74) Representative: Helbing, Jörg
(86) International application number: PCT/JP2005/020492
(87) International publication number: WO 2006/049312

(57) **Abstract**

The present invention provides therapeutic agents for neuropathic pain, the agents having excellent therapeutic effects on neuropathic pain, which is an intractable disorder. More specifically, the invention provides therapeutic agents for neuropathic pain comprising, as the active ingredient, an opioid receptor antagonist (particularly naloxone, naltrexone, naloxonazine, naltrindole, etc.), pharmaceutical compositions for treating neuropathic pain comprising an opioid receptor antagonist as the active ingredient, and a method for treating neuropathic pain using opioid receptor antagonists.

## Description

### TECHNICAL FIELD

The present invention relates to a therapeutic agent for neuropathic pain which has an excellent pain suppressing action against neuropathic pain, and to a method for treating neuropathic pain using such a therapeutic agent or the like.

### BACKGROUND ART

Neuropathic pain is pain caused by injury or dysfunction in the peripheral or central nervous system. It is intractable pain for which opioid receptor agonists such as morphine are not sufficiently effective. Disorders with associated neuropathic pain include disorders that exhibit hyperalgesic or allodynic symptoms, such as postherpetic neuralgia, trigeminal neuralgia, diabetic neuralgia, and persistent postoperative or posttraumatic pain.

Analgesics that have hitherto been used in conventional drug treatment are known to include centrally acting opioid receptor agonists such as morphine and non-steroidal anti-inflammatory drugs (NSAIDs) such as indomethacin. However, it is known that these analgesics generally have only a small effect on neuropathic pain, and that such effects by analgesics which work well for ordinary nociceptive pain (particularly narcotic analgesics) are especially small. Moreover, the inadequate analgesic effect by narcotic analgesics on neuropathic pain is regarded as a major characteristic of neuropathic pain. In some cases, the diagnosis of neuropathic pain is carried out using this characteristic.

Various factors are thought to be intricately involved in the onset of neuropathic pain. Up until now, known treatment modalities for neuropathic pain have include neurosurgical intervention such as nerve blocking and spinal epidural stimulation, and the lumbar intrathecal administration of drugs such as tricyclic antidepressants and baclofen. However, these methods of treatment are either not sufficiently effective or have side effects. As for external preparations, capsaicin cream, by depleting the pain-producing substance P that is released from the nerve endings and alleviating pain, has been reported to be effective for postherpetic neuralgia and postmastectomy pain syndrome. However, due in part to the fact that capsaicin causes burning pain, there are problems with the usefulness and safety of this medication. Hence, neuropathic pain is an intractable disorder for which an effective method of treatment has yet to be established.

Pain treatment involving the use of an opioid receptor agonist such as buprenorphine or nalbuphine together with a low dose of an opioid receptor antagonist such as naloxone is also known. Specifically, Japanese Patent Application (Published Japanese Translation of PCT International Publication) No. 2003-514013 describes the use of buprenorphine together with opioid receptor antagonists, and Japanese Patent Application (Published Japanese Translation of PCT International Publication) No. 2003-535833 describes the use of nalbuphine together with opioid receptor antagonists. Similarly, the use of an opioid alkaloid such as morphine or an opioid peptide together with a low dose of an opioid receptor antagonist is also known (Japanese Patent Application (Published Japanese Translation of PCT International Publication) No. H10-507740). The combination drugs described in these patent publications are characterized by the use of an opioid receptor agonist together with a low dose of an opioid receptor antagonist. The low dose of opioid receptor antagonist functions to increase the analgesic action of the opioid receptor agonist. In any case, these publications do not mention or suggest in any way that such drugs, when used together, are effective against neuropathic pain.

### DISCLOSURE OF THE INVENTION

As noted above, pharmaceuticals effective for treating neuropathic pain are not yet known. Hence, a desire exists for the development of such pharmaceuticals. In light of this, it is an object of the present invention to provide a novel therapeutic agent for neuropathic pain which is highly effective against this type of intractable pain.

The inventors have pursued research based on their own ideas for achieving this object, and, as a result found that opioid receptor antagonists such as naloxone exhibit a highly analgesic effect in an intractable neuropathic pain model. This discovery led ultimately to the present invention.

Accordingly, the present invention provides the following therapeutic agents for neuropathic pain, pharmaceutical compositions for treating neuropathic pain, methods for treating neuropathic pain and the like.
(1) A therapeutic agent for neuropathic pain, comprising an opioid receptor antagonist as an active ingredient.
(2) The therapeutic agent for neuropathic pain of (1) above, wherein the opioid receptor antagonist is a subtype-nonselective opioid receptor antagonist.
(3) The therapeutic agent for neuropathic pain of (2), wherein the subtype-nonselective opioid receptor antagonist is selected from the group consisting of naloxone, naltrexone, diprenorphine, β-chlornaltrexamine (β-CAN), buprenorphine, nalmefene, (9-[3-(cis-2,5-dimethyl-1-piperazinyl)propyl]carbazole dihydrochloride and pharmaceutically acceptable salts thereof.
(4) The therapeutic agent for neuropathic pain of (3) above, wherein the subtype nonselective opioid receptor antagonist is selected from the group consisting of naloxone, naltrexone and pharmaceutically acceptable salts thereof.
(5) The therapeutic agent for neuropathic pain of (1) above, wherein the opioid receptor antagonist is a subtype-selective opioid receptor antagonist.
(6) The therapeutic agent for neuropathic pain of (5) above, wherein the subtype-selective receptor antagonist is a µ subtype-selective opioid receptor antagonist.
(7) The therapeutic agent for neuropathic pain of (6) above, wherein the µ subtype-selective opioid receptor antagonist is selected from the group consisting of naloxonazine, CTAP (D-Phe-Cys-Tyr-D-Trp-Arg-Thr-Pen-Thr-NH₂), CTOP (D-Phe-Cys-Tyr-D-Trp-Om-Thr-Phe-Thr-NH₂), β-funaltrexamine (β-FNA), methocinnamox (M-CAM), cyprodime, 3-methoxynaltrexone and pharmaceutically acceptable salts thereof.
(8) The therapeutic agent for neuropathic pain of (7) above, wherein the µ subtype-selective opioid receptor antagonist is selected from naloxonazine and pharmaceutically acceptable salts thereof.
(9) The therapeutic agent for neuropathic pain of (5) above, wherein the subtype-selective receptor antagonist is a δ subtype-selective opioid receptor antagonist.
(10) The therapeutic agent for neuropathic pain of (9) above, wherein the δ subtype-selective opioid receptor antagonist is selected from the group consisting of naltriben, naltrindole, BNTX ((E)-7-benzylidenenaltrexone), DALCE ([D-Ala², Leu⁵, Cys⁶]-Enkephalin), 5'-NTII (naltrindole 5'-isothiocyanate), NTB (benzofuran analog of naltrindole), TIPP (Ψ) (H-Tyr-TicΨ-[CH₂NH]Phe-Phe-OH), ICI-174,864 (N,N-diallyl-Tyr-Aib-Aib-Phe-Leu) and pharmaceutically acceptable salts thereof.
(11) The therapeutic agent for neuropathic pain of (10) above, wherein the δ subtype-selective opioid receptor antagonist is selected from naltrindole and pharmaceutically acceptable salts thereof.
(12) The therapeutic agent for neuropathic pain of any of (1) to (11) above, wherein the neuropathic pain is one or more symptoms selected from the group consisting of postherpetic neuralgia, trigeminal neuralgia, diabetic neuralgia, cancer pain, persistent postoperative or posttraumatic pain, hyperalgia, allodynia, postthoracotomy pain, CRPS, pain associated with multiple sclerosis, AIDS, thalamic pain, paraplegic pain caused by myelopathy, anesthesia dolorosa and phantom limb pain.
(13) A pharmaceutical composition for treating neuropathic pain, comprising an opioid receptor antagonist and a pharmaceutically acceptable carrier.
(14) A method for treating neuropathic pain, comprising administering an effective amount of an opioid receptor antagonist to a mammal.
(15) Use of an opioid receptor antagonist for manufacturing a therapeutic agent for neuropathic pain.

The inventive therapeutic agent for neuropathic pain is effective for the treatment of neuropathic pain which exhibits symptoms such as postherpetic neuralgia, trigeminal neuralgia, diabetic neuralgia, cancer pain, persistent postoperative or posttraumatic pain, hyperalgia and allodynia. In particular, the naloxone, naltrexone and pharmaceutically allowable salts thereof that are preferably used as the active ingredient in the therapeutic agent of the invention have already undergone full clinical trials as therapeutic agents for other disorders and are currently sold commercially as prescription medications. Accordingly, therapeutic agents for neuropathic pain which include these active ingredients have the additional advantage that their safety for use in humans has been demonstrated.

### BRIEF DESCRIPTION OF THE DIAGRAMS

FIG. 1, which shows the experimental results from Example 1 of the invention, is a graph showing the change in the pain threshold to mechanical stimulation in hyperalgesic rats that have been intraperitoneally administered a low dose of naloxone.
FIG. 2, which shows the experimental results from Example 2, is a graph showing the change in pain threshold to thermal stimulation in hyperalgesic rats that have been intraperitoneally administered a low dose of naloxone.
FIG. 3, which shows the experimental results from Example 3, is a graph showing the change in pain threshold to mechanical stimulation in hyperalgesic rats that have been intraperitoneally administered a high dose of naloxone.
FIG. 4, which shows the experimental results from Example 4, is a graph showing the change in pain threshold to thermal stimulation in hyperalgesic rats that have been intraperitoneally administered a high dose of naloxone.
FIG. 5, which shows the experimental results from Example 5, is a graph showing the change in pain threshold to mechanical stimulation in hyperalgesic rats that have been intraperitoneally administered naltrexone.
FIG. 6, which shows the experimental results from Example 6, is a graph showing the change in pain threshold to thermal stimulation in hyperalgesic rats that have been intraperitoneally administered naltrexone.
FIG. 7, which shows the experimental results from Example 7, is a graph showing the change in pain threshold to mechanical stimulation in hyperalgesic rats that have been intraperitoneally administered naloxonazine.
FIG. 8, which shows the experimental results from Example 8, is a graph showing the change in pain threshold to thermal stimulation in hyperalgesic rats that have been intraperitoneally administered naloxonazine.
FIG. 9, which shows the experimental results from Example 9, is a graph showing the change in pain threshold to mechanical stimulation in hyperalgesic rats that have been intraperitoneally administered naltrindole.
FIG. 10, which shows the experimental results from Example 10, is a graph showing the change in pain threshold to thermal stimulation in hyperalgesic rats that have been intraperitoneally administered naltrindole.

### BEST MODE FOR CARRYING OUT THE INVENTION

The invention is described in more detail below.

The present invention provides therapeutic agents for neuropathic pain which comprises an opioid receptor antagonist as the active ingredient, pharmaceutical compositions for treating neuropathic pain which comprises an opioid receptor antagonist and a pharmaceutically acceptable carrier, and a method for treating neuropathic pain using an opioid receptor antagonist. Although various opioid receptor-specific antagonists such as naloxone have hitherto been known, the inventors have surprisingly made the original and unexpected discovery that such opioid receptor-specific antagonists per se produce a therapeutic effect against neuropathic pain. In particular, because opioid receptor agonists such as morphine have been customarily used as therapeutic agents for intractable pain, no one thought that opioid receptor antagonists such as naloxone would have some sort of analgesic effect against certain types of pain. The literature is thus devoid of any studies on the pain suppressing effects of opioid receptor antagonists alone in neuropathic pain models. This fact in itself demonstrates the originality of the present invention.

In the specification, "opioid receptor antagonist" refers to a substance having an antagonistic action on opioid receptors. Antagonistic actions on opioid receptors can be verified by a known technique, such as the method described in Pharmacol. Biochem. Behavior 74: 841 to 849 (2003). Opioid receptors are known to exist in at least three subtypes: µ-, δ-, and κ- (in addition, opioid receptor-like orphan receptors (ORL₁) have also been found). The opioid receptor antagonists of the invention include agents having antagonistic actions for at least one of these three types of receptors. In this specification, the term "therapy" or "treatment" generally means to effect an improvement in the symptoms of humans or mammals other than humans. The term "improvement" denotes cases where, compared with cases in which the therapeutic agent of the invention is not administered, the severity of a disorder is alleviated or does not worsen, and also encompasses therein the meaning of prophylaxis. In addition, the term "pharmaceutical composition" refers to a composition containing an active ingredient (e.g., naloxone) useful in the invention and excipients such as carriers that may be employed in preparing a pharmaceutical.

Opioid receptor antagonists that may used in the invention are not subject to any particular limitation, and include both subtype-nonselective opioid receptor antagonists and subtype-selective opioid receptor antagonists. Subtype-nonselective opioid receptor antagonists preferred for use in the invention include naloxone, naltrexone, diprenorphine, β-chlornaltrexamine, buprenorphine, nalmefene, (9-[3-(cis-2,5-dimethyl-1-piperazinyl)propyl]carbazole dihydrochloride and pharmaceutically acceptable salts thereof. Of these subtype-nonselective opioid receptor antagonists, naloxone and naltrexone are especially preferred. Naloxone is most preferred. Naloxone and naltrexone are known compounds having mutually similar structures. These nonselective opioid receptor antagonists are all known compounds cited in, for example, The Merck Index, 13th Edition (2001), a SIGMA-RBI Catalog (Cell Signaling and Neuroscience, pp. 740 to 742, 771 to 772 (2004 to 2005), and pharmacological textbooks (e.g., The Pharmacological Basis of Therapeutics, 9th Edition, McGraw-Hill). For example, entries for naloxone and naltrexone appear on pages 1140 to 1141 of the foregoing Merck Index, and include their chemical names, chemical structures, physicochemical properties, and major literature references.

µ Subtype-selective opioid receptor antagonists preferred for use in the invention include naloxonazine, CTAP, CTOP, β-funaltrexamine, methocinnamox, cyprodime, 3-methoxynaltrexone and pharmaceutically acceptable salts thereof. These µ subtype-selective opioid receptor antagonists are all known compounds cited in, for example, The Merck Index, 13th Edition (2001), a SIGMA-RBI Catalog (Cell Signaling and Neuroscience, pp. 740 to 742, 771 to 772 (2004 to 2005), and pharmacological textbooks (e.g., The Pharmacological Basis of Therapeutics, 9th Edition, McGraw-Hill). Of these µ subtype-selective opioid receptor antagonists, naloxonazine is especially preferred. δ Subtype-selective opioid receptor antagonists preferred for use in the invention include naltriben, naltrindole, BNTX, DALCE, 5'-NTII, NTB, TIPP (Ψ), ICI-174,864 and pharmaceutically acceptable salts thereof. These δ subtype-selective opioid receptor antagonists are all known compounds cited in, for example, The Merck Index, 13th Edition (2001), a SIGMA-RBI Catalog (Cell Signaling and Neuroscience, pp. 740 to 742, 771 to 772 (2004 to 2005), and pharmacological textbooks (e.g., The Pharmacological Basis of Therapeutics, 9th Edition, McGraw-Hill). Of these δ subtype-selective opioid receptor antagonists, naltrindole is especially preferred.

In the specification, the phrase "comprising, as the active ingredient, an opioid receptor antagonist" is used in a sense that entirely encompasses uses of compounds known to be opioid receptor antagonists and uses of such compounds in pharmaceutically acceptable forms (e.g., salts, esters, amides, hydrates or solvate forms thereof, and racemic mixtures or optically pure forms).

Accordingly, the compound used as the active ingredient in the invention may be either a free compound or a pharmaceutically acceptable salt. The reference herein to "salt" includes both acid salts and basic salts. Examples of acid salts include hydrochlorides, hydrobromides, hydroiodides, nitrates, sulfates, bisulfates, phosphates, acidic phosphates, acetates, lactates, citrates, acidic citrates, tartrates, bitartrates, succinates, maleates, fumarates, gluconates, aldarates, benzoates, methanesulfonates, ethanesulfonates, benzenesulfonates, p-toluenesulfonates and 1,1'-methylenebis(2-hydroxy-2-naphthoic acid) salts. Examples of basic salts include alkali metal salts such as sodium salts and potassium salts, alkaline earth metal salts such as calcium salts and magnesium salts, water-soluble amine addition salts such as ammonium salts and N-methylglucamine salts, lower alkanol ammonium salts, and salts derived from other organic amine bases that are pharmaceutically acceptable.

The therapeutic agents for neuropathic pain and compositions of the invention are effective for treating neuropathic pain. Illustrative examples of such neuropathic pain include postherpetic neuralgia, trigeminal neuralgia, diabetic neuralgia, cancer pain, persistent postoperative or posttraumatic pain, hyperalgia, allodynia, postthoracotomy pain, CRPS, pain associated with multiple sclerosis, AIDS, thalamic pain, paraplegic pain caused by myelopathy, anesthesia dolorosa and phantom limb pain. The inventive therapeutic agent for neuropathic pain is particularly effective for treating hyperalgia and allodynia.

No particular limitation is imposed on the dosage form of the inventive therapeutic agent for neuropathic pain. Administration may be carried out orally or parenterally. The opioid receptor antagonist serving as the active ingredient of the inventive therapeutic agent for neuropathic pain may be formulated alone, or formulated together with a pharmaceutically acceptable carrier or pharmaceutical excipients and furnished in the form of a pharmaceutical. In such cases, the opioid receptor antagonist serving as the active ingredient of the invention may be included within the pharmaceutical in an amount of from 0.1 to 99.9 wt%.

Pharmaceutically acceptable carriers or excipients that may be used include fillers, disintegrants, disintegrating aids, binders, lubricants, coatings, colors, dispersing agents, solvents, solubilizing agents, tonicity agents, pH modifiers, and stabilizers.

Preparations suitable for oral administration include powdered preparations, tablets, capsules, fine granules, granules, liquid preparations and syrups. For parenteral administration, various fillers such as microcrystalline cellulose, sodium citrate, calcium carbonate, dipotassium phosphate or glycine may be used together with a starch (preferably corn, potato or tapioca starch); any of various disintegrants such as arginic acid or some type of silicate double salt; and a fine granule-forming binder such as polyvinyl pyrrolidone, sucrose, gelatin or gum arabic. Moreover, lubricants such as magnesium stearate, sodium lauryl sulfate and talc are often very effective for tablet formation. It is also possible to use gelatin capsules filled with solid compositions of the same type. Substances that may be suitably used in connection with this include not only lactose, but also high-molecular-weight polyethylene glycols. For preparation as an aqueous suspension and/or elixir for oral administration, the active ingredient may be used together with any of various types of sweeteners or flavorings and colorants or dyes, as well as an optional emulsifier and/or a suspending agent, along with a diluting agent such as water, ethanol, propylene glycerol, glycerol or a combination thereof.

Illustrative examples of preparations suitable for parenteral administration include injections and suppositories. In the case of parenteral administration, the active ingredient of the invention may be dissolved in either sesame oil or peanut oil, or use may be made of a solution obtained by dissolving the active ingredient in a propylene glycol-water solution. If necessary, the aqueous solution may be suitably buffered (preferably to a pH of 8 or more), and it may be necessary to first render the liquid dilution isotonic. Such aqueous solutions are suitable for intravenous injection, and oleaginous solutions are suitable for intraarticular injections, intramuscular injection and subcutaneous injection. The manufacture of all of these solutions under aseptic conditions can easily be achieved by standard pharmaceutical manufacturing technology familiar to those conversant with the art. In addition, the active ingredients of the invention may be topically administered to the skin or the like. In such cases, in accordance with standard pharmaceutical practice, it is desirable to carry out topical administration in the form of a cream, jelly, paste or ointment.

No particular limitation is imposed on the dosage of the inventive therapeutic agent for neuropathic pain. A dosage suitable for the various conditions, such as the type of pain, the age and symptoms of the patient, the route of administration, the purpose of treatment, and the presence or absence of accompanying medications, may be selected. The dosage of the inventive therapeutic agent for neuropathic pain in an adult (with a body weight of, for example, 60 kg) may be, for example, from about 100 to about 25,000 mg, and preferably from about 150 to about 9,000 mg, per day. When administered as an injection, the daily dosage in an adult (with a body weight of, for example, 60 kg) may be, for example, from about 100 to about 5,000 mg, and preferably from about 180 to about 1,800 mg. These daily dosages may be divided into between two and four separate doses.

### EXAMPLES

Examples are given below to more fully illustrate the present invention, but are not intended to limit the scope of the invention.

### (Experimental Materials and General Experimental Method)

### (1) Model Animals

Use was made of a hyperalgesia model induced by complete ligation of the L5/L6 spinal nerves in five- to seven-week-old male rats as the experimental animals.

### (2) Study Groups

Mechanical stimulation tests were carried out using the Ugo Basile Model 37400 Dynamic Plantar Aesthesiometer, and thermal stimulation tests were carried out using a plantar thermal stimulation tester (Plantar Test 7370, Ugo Basile). The pain thresholds of the feet on each model animal were measured, and the animals were divided into groups having uniform pain thresholds as measured prior to administration on each day of the experiment. In mechanical stimulation, animals for which the model animal foot pain threshold was 8.0 g or more were excluded from the tests. In thermal stimulation, animals for which the pain threshold for the model foot was 10 seconds or more were excluded from the tests.

### (3) Preparation of Test Substance

The required amount of the test substance was weighed out and dissolved in physiological saline as the medium. A 2 mg/ml solution was prepared as the highest concentration mixture in the low dosage study, and a 24 mg/ml solution was prepared as the highest concentration mixture in the high dosage study. Solutions at the various concentrations were all prepared at the time of use by suitably diluting these highest concentration mixtures.

### (4) Method of Administration

Although the object here is to ascertain the direct effect of the test substance on the spinal cord, because the substance has been confirmed to pass through the blood-brain barrier, intraperitoneal administration, which is a simple method of administration, was employed. Using the syringe barrel and needle, a volume of 5 ml/kg was administrated intraperitoneally.

### Example 1

### Mechanical Stimulation Method - Low Dose Study:

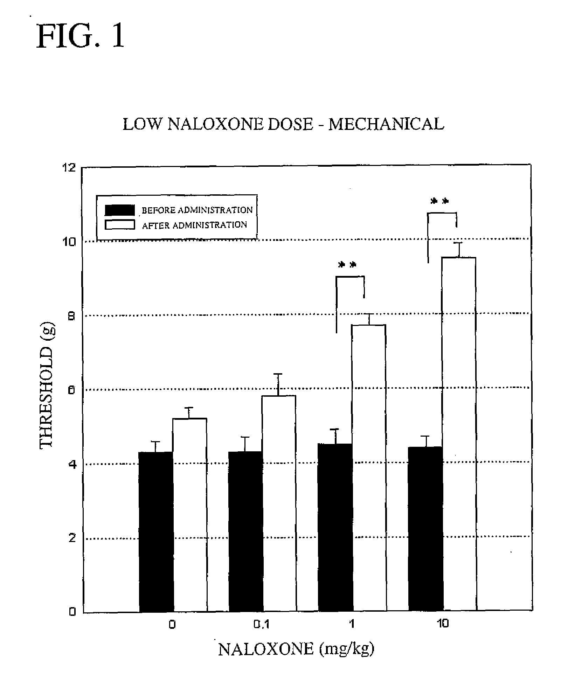

Groups of five male rats (333.7 to 414.2 g) in which the hyperalgesia model had been induced were used. An aesthesiometer set to a maximum pressure of 15.0 g and to a time until the maximum pressure is reached of 20 seconds was used to measure the left plantar pain threshold before naloxone administration, and 20 minutes, 40 minutes and 60 minutes after administration. The results are shown in FIG. 1. In the figure, "**" shows the significant difference at P<0.01 based on Dunnett's multiple comparison test, and "*" shows the significant difference at P<0.05 based on Dunnett's multiple comparison test.

As shown in FIG. 1, in a control group administered physiological saline, the maximum pain threshold following administration was 5.2 g. By contrast, in the groups administered naloxone, (a) at a dose of 0.1 mg/kg, the maximum threshold following administration was 5.8 g; (b) at a dose of 1 mg/kg, the maximum threshold following administration was 7.7 g; and (c) at a dose of 10 mg/kg, the maximum threshold following administration was 9.5 g. The pain threshold thus rose significantly with the administration of 1 mg and 10 mg of naloxone, demonstrating an analgesic effect against neuropathic pain.

### Example 2

### Thermal Stimulation Method - Low Dose Study:

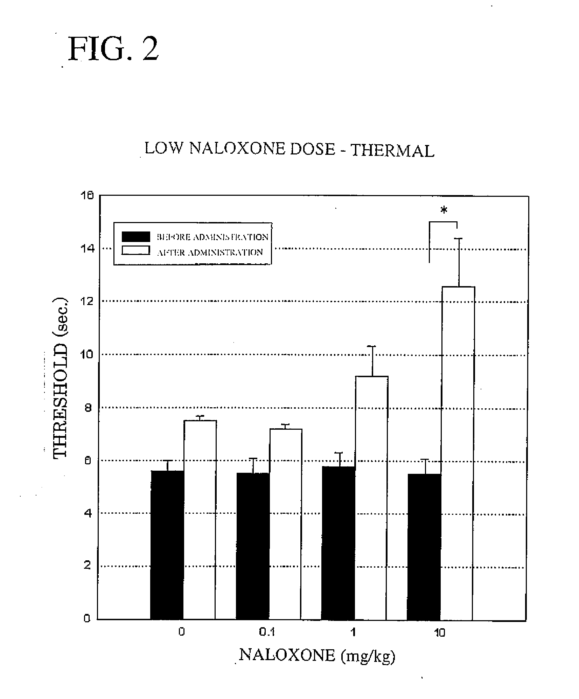

Groups of five male rats (356.7 to 444.0 g) in which the hyperalgesia model had been induced were used. A plantar thermal stimulation tester set to a thermal stimulation intensity of 35 was used to measure the left plantar pain threshold before naloxone administration, and 20 minutes, 40 minutes and 60 minutes after administration. The results are shown in FIG. 2.

As shown in FIG. 2, in a control group administered physiological saline, the maximum pain threshold following administration was 7.5 seconds. By contrast, in the groups administered naloxone, (a) at a dose of 0.1 mg/kg, the maximum threshold following administration was 7.2 seconds; (b) at a dose of 1 mg/kg, the maximum threshold following administration was 9.2 seconds; and (c) at a dose of 10 mg/kg, the maximum threshold following administration was 12.6 seconds. The pain threshold thus rose significantly with the administration of 10 mg of naloxone, demonstrating an analgesic effect against neuropathic pain.

### Example 3

### Mechanical Stimulation Method - High Dose Study:

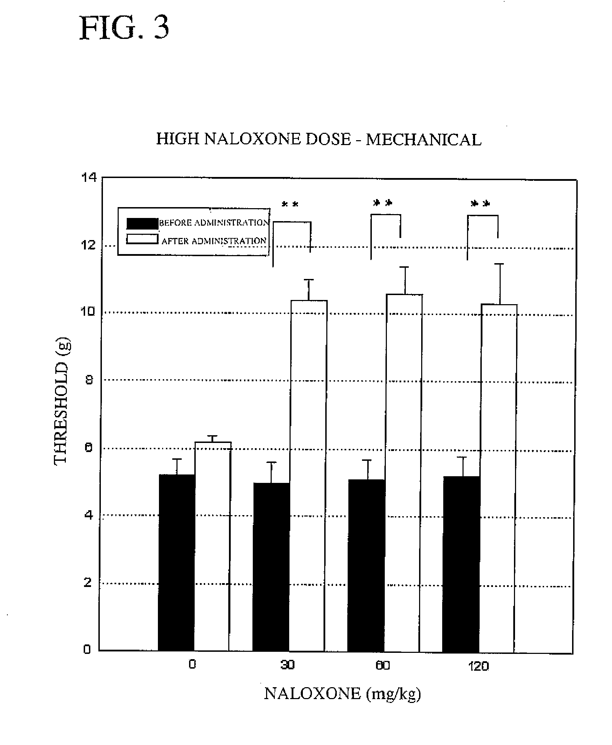

Groups of five male rats (372.0 to 459.5 g) in which the hyperalgesia model had been induced were used. An aesthesiometer set to a maximum pressure of 15.0 g and to a time until the maximum pressure is reached of 20 seconds was used to measure the left plantar pain threshold before naloxone administration, and 20 minutes, 40 minutes and 60 minutes after administration. The results are shown in FIG. 3.

As shown in FIG. 3, in a control group administered physiological saline, the maximum pain threshold following administration was 6.2 g. By contrast, in the groups administered naloxone, (a) at a dose of 30 mg/kg, the maximum threshold following administration was 10.4 g; (b) at a dose of 60 mg/kg, the maximum threshold following administration was 10.6 g; and (c) at a dose of 120 mg/kg, the maximum threshold following administration was 10.3 g. The pain threshold thus rose significantly with the administration of naloxone, demonstrating an analgesic effect against neuropathic pain. This effect reached its peak at a dose of 60 mg/kg. In the above-described hyperalgesia model, allodynia, a condition in which normally non-painful tactile stimuli evoke a pain sensation, arose, dramatically lowering the pain threshold. However, with the intraperitoneal administration of naloxone, the pain threshold rose dose-dependently, demonstrating an improvement in hyperalgesia.

### Example 4

### Thermal Stimulation Method - High Dose Study:

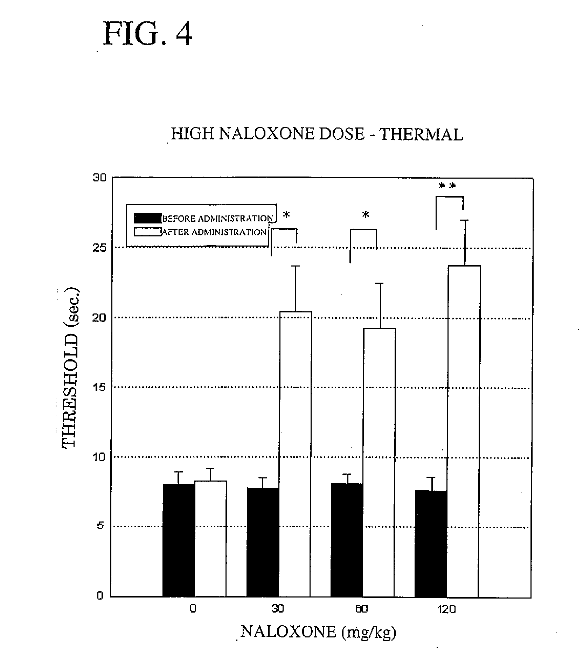

Groups of five male rats (400.3 to 499.9 g) in which the hyperalgesia model had been induced were used. A plantar thermal stimulation tester set to a thermal stimulation intensity of 35 was used to measure the left plantar pain threshold before naloxone administration, and 20 minutes, 40 minutes and 60 minutes after administration. The results are shown in FIG. 4.

As shown in FIG. 4, in a control group administered physiological saline, the maximum pain threshold following administration was 8.3 seconds. By contrast, in the groups administered naloxone, (a) at a dose of 30 mg/kg, the maximum threshold following administration was 20.4 seconds; (b) at a dose of 60 mg/kg, the maximum threshold following administration was 19.3 seconds; and (c) at a dose of 120 mg/kg, the maximum threshold following administration was 23.8 seconds.

As in Example 3, the pain threshold thus rose significantly with the administration of naloxone, demonstrating an analgesic effect against neuropathic pain. This effect reached its peak at a dose of 30 mg/kg. In the above-described hyperalgesia model, allodynia, a condition in which normally non-painful tactile stimuli evoke a pain sensation, arose, dramatically lowering the pain threshold. However, with the intraperitoneal administration of naloxone, the pain threshold rose dose-dependently, demonstrating an improvement in hyperalgesia.

### Example 5

### Mechanical Stimulation Method:

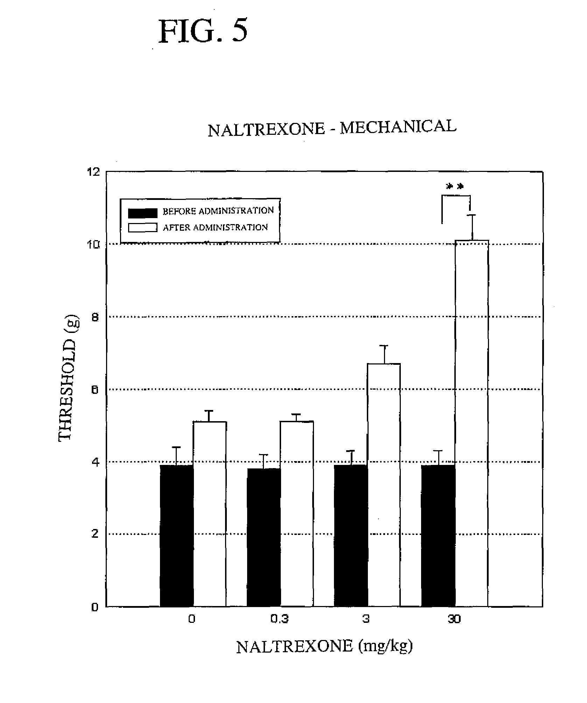

Groups of five male rats (295.6 to 356.6 g) in which the hyperalgesia model had been induced were used. An aesthesiometer set to a maximum pressure of 15.0 g and to a time until the maximum pressure is reached of 20 seconds was used to measure the left plantar pain threshold before naltrexone administration, and 20 minutes, 40 minutes and 60 minutes after administration. The results are shown in FIG. 5.

As shown in FIG. 5, in a control group administered physiological saline, the maximum pain threshold following administration was 5.1 g. By contrast, in the groups administered naltrexone, (a) at a dose of 0.3 mg/kg, the maximum threshold following administration was 5.1 g; (b) at a dose of 3 mg/kg, the maximum threshold following administration was 6.7 g; and (c) at a dose of 30 mg/kg, the maximum threshold following administration was 10.1 g. The pain threshold thus rose significantly with the administration of 30 mg/kg of naltrexone, demonstrating an analgesic effect against neuropathic pain.

### Example 6

### Thermal Stimulation Method:

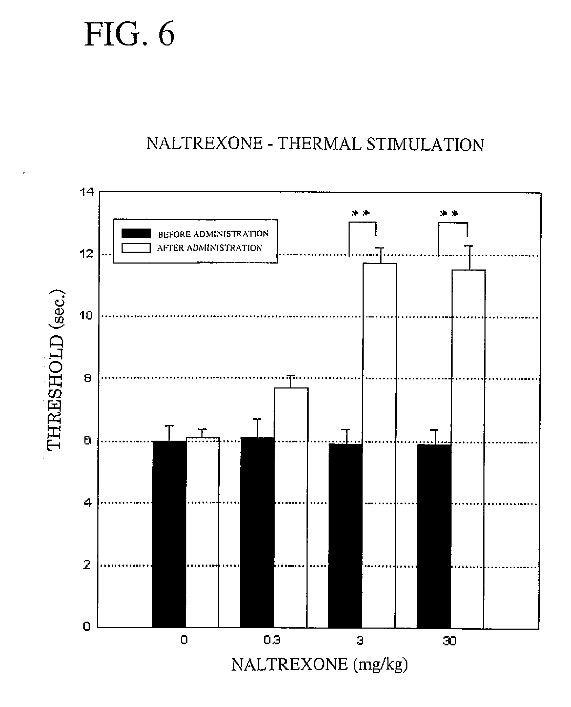

Groups of five male rats (324.8 to 399.0 g) in which the hyperalgesia model had been induced were used. A plantar thermal stimulation tester set to a thermal stimulation intensity of 35 was used to measure the left plantar pain threshold before naltrexone administration, and 20 minutes, 40 minutes and 60 minutes after administration. The results are shown in FIG. 6.

As shown in FIG. 6, in a control group administered physiological saline, the maximum pain threshold following administration was 6.1 seconds. By contrast, in the groups administered naltrexone, (a) at a dose of 0.3 mg/kg, the maximum threshold following administration was 7.7 seconds; (b) at a dose of 3 mg/kg, the maximum threshold following administration was 11.7 seconds; and (c) at a dose of 30 mg/kg, the maximum threshold following administration was 11.5 seconds. The pain threshold thus rose significantly with the administration of 3 and 30 mg/kg of naltrexone, demonstrating an analgesic effect against neuropathic pain.

### Example 7

### Mechanical Stimulation Method:

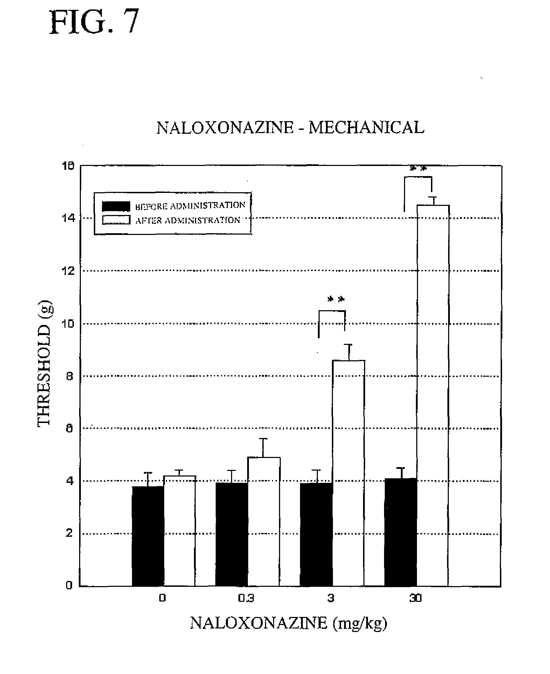

Groups of five male rats (257.7 to 340.3 g) in which the hyperalgesia model had been induced were used. An aesthesiometer set to a maximum pressure of 15.0 g and to a time until the maximum pressure is reached of 20 seconds was used to measure the left plantar pain threshold before naloxonazine administration, and 20 minutes, 40 minutes and 60 minutes after administration. The results are shown in FIG. 7.

As shown in FIG. 7, in a control group administered physiological saline, the maximum pain threshold following administration was 4.2 g. By contrast, in the groups administered naloxonazine, (a) at a dose of 0.3 mg/kg, the maximum threshold following administration was 4.9 g; (b) at a dose of 3 mg/kg, the maximum threshold following administration was 8.6 g; and (c) at a dose of 30 mg/kg, the maximum threshold following administration was 14.5 g. The pain threshold thus rose significantly with the administration of 3 and 30 mg/kg of naloxonazine, demonstrating an analgesic effect against neuropathic pain.

### Example 8

### Thermal Stimulation Method:

Groups of five male rats (307.0 to 393.1 g) in which the hyperalgesia model had been induced were used. A plantar thermal stimulation tester set to a thermal stimulation intensity of 35 was used to measure the left plantar pain threshold before naloxonazine administration, and 20 minutes, 40 minutes and 60 minutes after administration. The results are shown in FIG. 2.

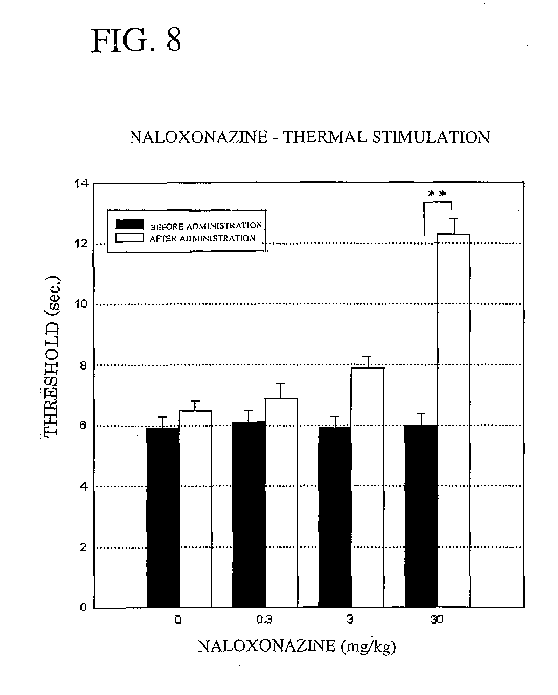

As shown in FIG. 8, in a control group administered physiological saline, the maximum pain threshold following administration was 6.5 seconds. By contrast, in the groups administered naloxonazine, (a) at a dose of 0.3 mg/kg, the maximum threshold following administration was 6.9 seconds; (b) at a dose of 3 mg/kg, the maximum threshold following administration was 7.9 seconds; and (c) at a dose of 30 mg/kg, the maximum threshold following administration was 12.3 seconds. The pain threshold thus rose significantly with the administration of 30 mg/kg of naloxonazine, demonstrating an analgesic effect against neuropathic pain.

### Example 9

### Mechanical Stimulation Method:

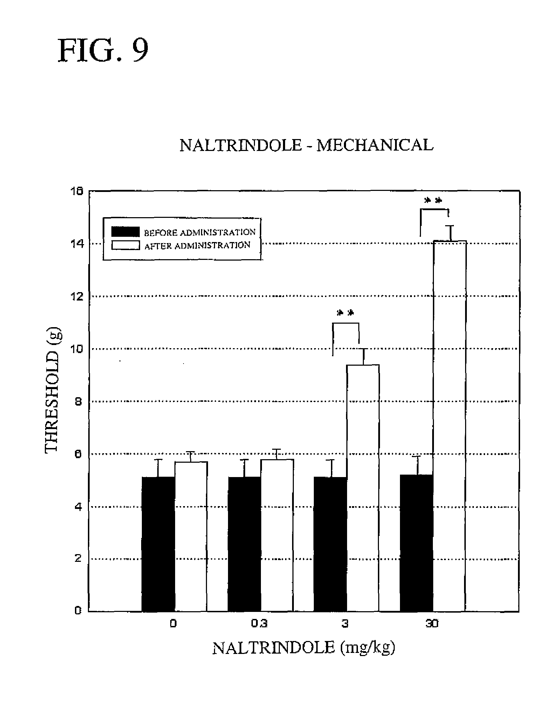

Groups of five male rats (349.2 to 450.4 g) in which the hyperalgesia model had been induced were used. An aesthesiometer set to a maximum pressure of 15.0 g and to a time until the maximum pressure is reached of 20 seconds was used to measure the left plantar pain threshold before naltrindole administration, and 20 minutes, 40 minutes and 60 minutes after administration. The results are shown in FIG. 9.

As shown in FIG. 9, in a control group administered physiological saline, the maximum pain threshold following administration was 5.7 g. By contrast, in the groups administered naltrindole, (a) at a dose of 0.3 mg/kg, the maximum threshold following administration was 5.8 g; (b) at a dose of 3 mg/kg, the maximum threshold following administration was 9.4 g; and (c) at a dose of 30 mg/kg, the maximum threshold following administration was 14.1 g. The pain threshold thus rose significantly with the administration of 3 and 30 mg/kg of naltrindole, demonstrating an analgesic effect against neuropathic pain.

### Example 10

### Thermal Stimulation Method:

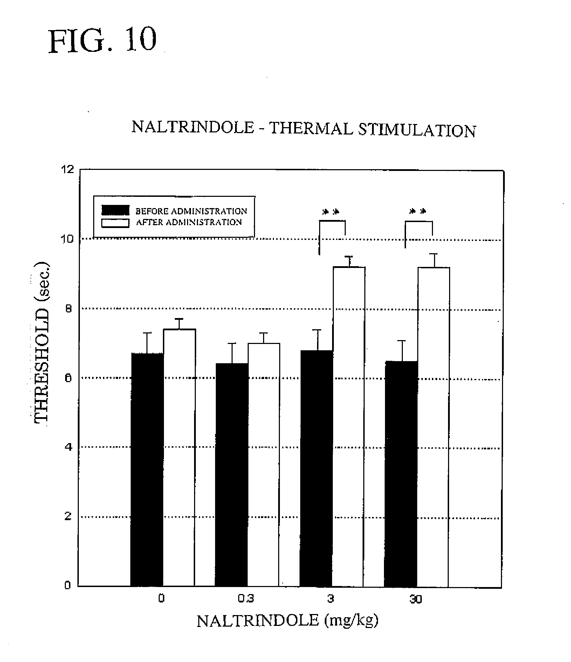

Groups of five male rats (365.3 to 437.2 g) in which the hyperalgesia model had been induced were used. A plantar thermal stimulation tester set to a thermal stimulation intensity of 35 was used to measure the left plantar pain threshold before naltrindole administration, and 20 minutes, 40 minutes and 60 minutes after administration. The results are shown in FIG. 10.

As shown in FIG. 10, in a control group administered physiological saline, the maximum pain threshold following administration was 7.4 seconds. By contrast, in the groups administered naltrindole, (a) at a dose of 0.3 mg/kg, the maximum threshold following administration was 7.0 seconds; (b) at a dose of 3 mg/kg, the maximum threshold following administration was 9.2 seconds; and (c) at a dose of 30 mg/kg, the maximum threshold following administration was 9.2 seconds. The pain threshold thus rose significantly with the administration of 3 and 30 mg/kg of naltrindole, demonstrating an analgesic effect against neuropathic pain.

### Discussion:

The above examples demonstrate, first of all, that subtype-nonselective opioid receptor antagonists are effective for treating neuropathic pain. Next, to determine whether such effects are specific to certain opioid receptor subtypes, the effects obtained using a µ selective opioid receptor antagonist and the effects obtained using a δ selective opioid receptor antagonist were investigated. The results showed that both have analgesic actions. Therefore, it became apparent that there exist at least two mechanisms for analgesic action by opioid receptor antagonists. That is, it was found that subtype-nonselective opioid receptor antagonists such as naloxone inhibit both µ receptors and δ receptors, thereby triggering analgesic effects by influencing the downstream systems of each. By contrast, µ selective opioid receptor antagonists specifically inhibit µ receptors, triggering analgesic effects by influencing only the downstream system of µ receptors, and δ selective opioid receptor antagonists specifically inhibit δ receptors, triggering analgesic effects by influencing only the downstream system of δ receptors,

### INDUSTRIAL APPLICABILITY

As explained above, the opioid receptor antagonist-comprising therapeutic agents for neuropathic pain of the invention have actions that ameliorate neuropathic pain symptoms because of a variety of causes, and can thus be effectively used for treating neuropathic pain. Because the preferred active ingredients in the inventive therapeutic agents for neuropathic pain, including some opioid receptor antagonists (e.g., naloxone, naltrexone, diprenorphine) now serving as therapeutic agents for other disorders and symptoms, have already successfully passed through clinical trials and are currently in use as prescription medications, there is the added advantage that these active ingredients have already been confirmed to be safe in patients.

## Claims

1. A therapeutic agent for neuropathic pain, comprising an opioid receptor antagonist as an active ingredient.

2. The therapeutic agent for neuropathic pain of claim 1, wherein the opioid receptor antagonist is a subtype-nonselective opioid receptor antagonist.

3. The therapeutic agent for neuropathic pain of claim 2, wherein the subtype-nonselective opioid receptor antagonist is selected from the group consisting of naloxone, naltrexone, diprenorphine, β-chlornaltrexamine, buprenorphine, nalmefene, (9-[3-(cis-2,5-dimethyl-1 -piperazinyl)propyl]carbazole dihydrochloride and pharmaceutically acceptable salts thereof.

4. The therapeutic agent for neuropathic pain of claim 3, wherein the subtype-nonselective opioid receptor antagonist is selected from the group consisting of naloxone, naltrexone and pharmaceutically acceptable salts thereof.

5. The therapeutic agent for neuropathic pain of claim 1, wherein the opioid receptor antagonist is a subtype-selective opioid receptor antagonist.

6. The therapeutic agent for neuropathic pain of claim 5, wherein the subtype-selective receptor antagonist is a µ subtype-selective opioid receptor antagonist.

7. The therapeutic agent for neuropathic pain of claim 6, wherein the µ subtype-selective opioid receptor antagonist is selected from the group consisting of naloxonazine, CTAP, CTOP, β-funaltrexamine, methocinnamox, cyprodime, 3-methoxynaltrexone and pharmaceutically acceptable salts thereof.

8. The therapeutic agent for neuropathic pain of claim 7, wherein the µ subtype-selective opioid receptor antagonist is selected from naloxonazine and pharmaceutically acceptable salts thereof.

9. The therapeutic agent for neuropathic pain of claim 5, wherein the subtype-selective receptor antagonist is a δ subtype-selective opioid receptor antagonist.

10. The therapeutic agent for neuropathic pain of claim 9, wherein the δ subtype-selective opioid receptor antagonist is selected from the group consisting of naltriben, naltrindole, BNTX, DALCE, 5'-NTII, NTB, TIPP (Ψ), ICI-174,864 and pharmaceutically acceptable salts thereof.

11. The therapeutic agent for neuropathic pain of claim 10, wherein the δ subtype-selective opioid receptor antagonist is selected from naltrindole and pharmaceutically acceptable salts thereof.

12. The therapeutic agent for neuropathic pain of any of claims 1 to 11, wherein the neuropathic pain is one or more symptoms selected from the group consisting of postherpetic neuralgia, trigeminal neuralgia, diabetic neuralgia, cancer pain, persistent postoperative or posttraumatic pain, hyperalgia, allodynia, postthoracotomy pain, CRPS, pain associated with multiple sclerosis, AIDS, thalamic pain, paraplegic pain caused by myelopathy, anesthesia dolorosa and phantom limb pain.

13. A pharmaceutical composition for treating neuropathic pain, comprising an opioid receptor antagonist and a pharmaceutically acceptable carrier.

14. A method for treating neuropathic pain, comprising administering an effective amount of an opioid receptor antagonist to a mammal.

15. Use of an opioid receptor antagonist for manufacturing a therapeutic agent for neuropathic pain.
